# EUROPEAN PATENT APPLICATION

(11) **EP 4 331 363 A1**
(43) Date of publication of application: **06.03.2024**
(21) Application number: 21938151.4
(22) Date of filing: 30.04.2021
(51) Int. Cl.: A01N 63/22, A01N 65/00, C05F 11/08

(54) **ACTIVATION OF BIONEMATICIDE AND PLANT GROWTH PROMOTING FUNCTIONS IN A BIOTECHNOLOGICAL SOLUTION**

(71) Applicant: Total Biotecnologia Indústria e Comércio S.A., 81460-020 Curitiba - PR (BR)
(72) Inventor: FUKAMI, Josiane, 81460-020 Curitiba - PR (BR); GOMES, Douglas Fabiano, 81460-020 Curitiba - PR (BR); MARCOLINA GOMES, Juliana, 81460-020 Curitiba - PR (BR); HIPOLITO DE ASSIS FILHO, Jonas, 81460-020 Curitiba - PR (BR)
(74) Representative: Regimbeau
(86) International application number: PCT/BR2021/050180
(87) International publication number: WO 2022/226608

(57) **Abstract**

The present invention relates to the potentiating effect of the association of botanical extract and *Bacillus* ssp. as a bionematicide agent and its surprising potential effect in promoting plant growth in a single product achieved by a distinguished industrial process. The industrial process of inducing the formation of *Bacillus* spp. endospores by osmotic stress of vegetative cells in a culture medium during bacterial growth is what allows the mixing of *Bacillus* spp. and botanical extract, since there is an incompatibility between the *Bacillus* species themselves and the botanical extract, both in the vegetative phase of the microorganism. Finally, synergy between the active ingredients, microbial cells and allicin, activate the bionematicide and growth-promoting function of the disclosed biotechnological composition.

## Description

### TECHNICAL FIELD OF THE INVENTION

The present invention is related to agricultural compositions comprising a combination of two or more bacterial species from the genus *Bacillus* and botanical extract comprising allicin as an active ingredient, which exhibits a bionematicide and plant growth promoting effect. The disclosed invention contemplates the process of preparing the compositions and agricultural application thereof.

### BACKGROUND OF THE INVENTION

*Bacillus* bacteria are Gram-positive microorganisms that are widely present in nature and can produce large amounts of enzymes and a wide range of antibiotics, making them excellent tools for agricultural use to promote plant growth and biological management of pathogens, including phytonematodes. Moreover, they have the ability to form resistance structures in abiotic stress conditions designated as endospores, increasing their survival in unfavorable environments and, when industrially induced, they ensure product stability for long periods of storage.

They are widely known for their beneficial effects, especially in plants of agronomic importance, whether for growth promotion or even as biodefensive agents against phytonematodes, that is, in favorable environments they are no longer in the resistance form (endospores) and assume the vegetative phase, triggering its mechanisms of action, such as the formation of biofilm and synthesis of metabolites having nematicide effect.

Another very important tool in the management of nematodes is the use of botanical extracts, such as garlic extracts, which act mainly via a "shock effect", that is, due to the nematostatic contacting effect that results in the target's death. From among the biotic factors that limit the crop yield, attack of pests, such as nematodes, is undoubtedly one of the most impactful for Brazilian agribusiness. According to Embrapa Soja, the damage caused by nematodes in Brazil is estimated at R$ 16.5 billion (US$ 3.7 billion), only in soybean crops and it is reported that the main agricultural crops grown in the country are susceptible to phytopathogens (Embrapa Soja, 2020). The edaphoclimatic characteristics of Brazil provide ideal conditions for the reproduction and feeding of the pest and, after having established themselves in a field, the nematodes are difficult to eradicate, which makes management tools essential. Gall nematodes (*Meloidogyne* spp.) and root lesion nematodes (*Pratylenchus* spp.) are among the main phytonematode species of agricultural relevance.

One of the active ingredients in garlic extract is allicin, a compound having broad antimicrobial activity even at low concentrations (µM), both against Gram-negative and Gram-positive bacteria, including the *Bacillus* genus (Leontiev et al., 2018). Therefore, it is common sense that the combination of garlic botanical extract with bacteria of the genus *Bacillus* would result in incompatibility, representing a technical challenge to be solved. Such an incompatibility between garlic extract and *Bacillus* is recognized by the scientific community and a described example is the inhibition of *Bacillus cereus* when in contact with 3%, 5% and 10% of said extract (Zahira et al., 1982).

In addition, there are reports relative to the incompatibility between the vegetative cells of different *Bacillus* species, which was evidenced in *in vitro* tests carried out with *B. pumilus* C116 and *Bacillus thuringiensis* subsp. *kenyae* C25 (Gomes et al., 2003). However, it is not a rule for *in vivo* assays, since the mixture of different *Bacillus* species can increase the suppression of diseases (De Boer, 1999). Duffy et al. (1996) have reported that co-inoculation of different microorganisms acts on different regions of the roots and hence it does not inhibit the action of each microorganism, or because the production of secondary inhibitory compounds takes place in the stationary phase rather than the initial stages of colonization.

Therefore, there is a need in the art for agricultural compositions comprising a combination of viable cells from two or more bacteria species of the genus *Bacillus* and a botanical extract comprising allicin.

### BRIEF DESCRIPTION OF THE PRESENT INVENTION

The present invention surprisingly solves the issue of incompatibility between two or more bacterial species of the genus *Bacillus* as well as with a botanical extract comprising allicin through the formulation of compositions using essentially endospores of *Bacillus* bacteria. The use of *Bacillus* bacterial endospores in accordance with the present invention enables the preparation of a unique and stable formulation, with prolonged shelf life and sustained activity of individual active ingredients, being capable of promoting a surprising effect on the control of phytonematode and on the promotion of plant growth.

In addition, the compositions of the present invention optionally further comprise a formulation that promotes germination of endospores in metabolically active forms only when applied in the field, where bacteria are able to play their respective biological roles without any prejudice between them. Among these inducers, the solution contains free amino acids, carbon sources and metabolites of microbial origin, which accelerate the biological activation of the endospores without depending on stochastic factors.

Thus, the present invention provides agricultural compositions comprising endospores of two or more species of *Bacillus* bacteria and a botanical extract comprising allicin, a method of preparation and uses thereof, as well as methods of controlling nematodes in plant crops and of promoting plant growth.

### BRIEF DESCRIPTION OF THE FIGURES

**Figure 1****.** Compatibility assay between *Bacillus velezensis* and *Bacillus amyloliquefaciens* in Petri dishes.
**Figure 2****.** Stability between cells of three *Bacillus* species in their two forms, namely, vegetative and resistance (endospores) forms, in the presence of garlic botanical extract after 24 and 48 hours of the mixture formulation.
**Figure 3****.** Nematicide effect of the botanical extract associated with three *Bacillus* species after a 24- and 48-hour exposure of nematodes.
**Figure 4****.** Synergistic effect of *Bacillus* spp. and botanical extracts to promote maize growth.
**Figure 5****.** Phenotype of maize plants treated with *Bacillus* spp. and botanical extracts. T1 = control; T2 = 3 *Bacillus* spp.; T3 = Botanical Extract; T4 = 3 *Bacillus* spp. + Botanical Extract.

### DETAILED DESCRIPTION OF THE PRESENT INVENTION

In a first aspect, the present invention provides an agricultural composition comprising endospores of two or more bacterial species of the genus *Bacillus* and a botanical extract comprising allicin. In a preferred aspect, the agricultural composition comprises endospores of three or more bacterial species of the genus *Bacillus.* Optionally, an agricultural composition according to the present invention further comprises components that promote germination of endospores in metabolically active forms only when applied in the field.

The use of *Bacillus* bacterial endospores in accordance with the present invention enables the preparation of a unique and stable formulation, with prolonged shelf life and sustained activity of individual active ingredients. Thus, an agricultural composition according to the present invention has a surprising and strong nematicide effect as well as promotes plant growth. The observed nematicide effect is greater than that presented by the separate components, bacterial cells and the botanical extract.

Species of the genus *Bacillus* being useful in the context of the present invention comprise, but are not limited to B. *velezensis, B. amyloliquefaciens, B. thuringiensis, B. megaterium, B. subtilis, B. firmus, Paenibacillus macerans, B. licheniformis*. Preferably, bacterial species of the genus *Bacillus* are selected from the group consisting of B. *velezensis* (Adetomiwa and Olubukola, 2019; Rabbee et al., 2019) , B. *amyloliquefaciens* (ldris et al., 2007; Liu et al., 2013; Xu et al., 2013) and *B. thuringiensis* (Bravo, Gill, and Soberón, 2007; Sansinenea, 2012; Schünemann, Knaak and Fiuza, 2014). As will be understood by the person skilled in the art, different strains of the aforementioned *Bacillus* species may be used.

Botanical extracts are rich in natural biologically active compounds having great potential for biotechnological application. Botanical extracts comprising compounds having bionematicide activity, such as allicin, which are useful in the context of the present invention comprise, but are not limited to, garlic extract (*Allium sativum*)*,* melaleuca (*Melaleuca alternifolia*)*,* neem (*Azadirachta indica*)*,* clove (*Syzygium aromaticum* ) . Preferably, a botanical extract comprising allicin is extracted mainly from garlic bulbs (*Allium sativum*) as it has a higher concentration of the active ingredient. These bioactive compounds derive from different extraction processes, from solvents having different polarities, mainly generating alcoholic extracts, aqueous or hydrolate extracts and essential oils. The main allicin extractors are: water, when ready to use; or through ethanolic solvents, since they are more efficient in extracting the active ingredient. In both methods, extracts are passed through a filtration process, and in some cases the extract can follow the step of allicin concentration. A botanical extract comprising allicin according to the present invention can be obtained by the method as described in Zahira et al., 1982; Curtis et al., 2004 and Fujisawa et al., 2008 incorporated herein in their entirety by reference.

Components that promote endospore germination in metabolically active forms only when applied in the field in the context of the present invention comprise, but are not limited to, carbon sources such as glucose, sucrose, maltose, cane molasses, nitrogen sources, including yeast extract, bacteriological peptone, free amino acids, including glutamic acid, tryptophan and glycine and, finally, metabolic extracts of microbial origin, including those rich in exopolysaccharides (EPS). A composition of the present invention may optionally contain additives, or agriculturally acceptable carriers, such as additives for treating seeds and sowing furrows, cell protectors for spray tanks, soil conditioners, grout adjuvants, polymers for seed coatings, polymers for coating granules of chemical or organic fertilizers, solid fertilizers, liquid fertilizers used in formulations for foliar products, endospore activators, among other purposes.

A composition according to the present invention can be packaged in a suitable packaging known in the art. Preferably a bag and/or plastic bottles can be used without the need for oxygen exchange of the final product. Packaging of the product of the present invention eliminates the need for light protection, as it is photostable. Preferably, the packaged volume can contain about 1 - 20 L and can be stored in refrigerated environments, or at room temperature, comprising the range of about 10 to 35 °C.

The present invention further provides a process for producing an agricultural composition comprising the steps of:
(a) fermentation of microbial cultures of two or more species of the genus *Bacillus;*
(b) imposing osmotic stress during or after the fermentation process; and
(c) formulation of an agricultural composition comprising the mixture of bacterial cells with the botanical extract comprising allicin in the same container.

The present invention also provides additional parameters for the fermentation of *Bacillus* cells, and cell sporulation, such as parameters of pressure, temperature, oxygenation (air volume and stirring) and culture media, making it possible for one to achieve the *Bacillus* endospores used in the preparation of an agricultural composition according to the present invention.

As will be understood by the person skilled in the art, different fermentation parameters and composition of the culture medium can be combined in the present invention.

In a preferred embodiment according to the present invention, batch fermentation of the culture of different *Bacillus* takes place for about 24 to 168 hours.

In a preferred embodiment, the method of the present invention comprises the sequential expansion (scheduling) of *Bacillus* spp. Cultures for inoculation of the fermentation culture. Preferably, sequential expansion starts at volumes of 100 mL, which serves as an inoculum for about 1 L. This, in turn, is inoculated in about 10 L, which are, then, inoculated in 180L tanks which are finally transferred to reactors of about 2,000 L.

In a preferred embodiment, *Bacillus* species are expanded in flasks of about 100 mL by incubation on an orbital shaker at about 80 rpm at about 200 rpm. Incubation time is preferably of from about 8 hours to about 24 hours. Preferably, *Bacillus* species are then grown in stainless steel flasks containing about 1 L of culture medium. The incubation time is preferably of about 8 to about 48 hours with an air flow rate of about 0.25 Nm³/h to about 1.0 Nm³/h (= 4.16 - 16.67 vvm).

In a preferred embodiment, the air flow rate of stainless-steel flasks containing about 10 L is, preferably, from about 0.25 to about 1.5 Nm³/h (= 0.41 - 2.5 vvm) and the incubation time is preferably about 8 hours to about 48 hours.

In a preferred embodiment, the culture temperature for multiplication of the three *Bacillus* species according to the present invention is about 22 °C to about 38 °C.

In a preferred embodiment, different *Bacillus* species are inoculated separately in the production scheduling up to 180L and mixed in the 2,000L fermenters as described for the present invention. To this end, in a preferred embodiment, after cultivation in two stainless steel flasks of about 1 L, said flasks are inoculated into two other stainless-steel flasks of about 10 L and then transferred to tanks containing about 180 L of specific culture medium for each microorganism, with the addition of a stainless steel flask containing about 5 L of the solution of endospore-forming salts for *Bacillus* spp. (Table 4), incubated for about 24 to about 168 hours. The air flow rate is preferably from about 2.0 to about 15.0 Nm³/h (= 0.16 - 1.25 vvm).

In a preferred embodiment, the step of mixing the different *Bacillus* species and botanical extract comprising allicin at a concentration of about 1% to about 30% is carried out at a temperature of about 22 °C to about 38 °C. The air flow rate is preferably about 1.0 Nm³/h and about 2.5 Nm³/h (= 0.0085 - 0.021 vvm). The pressure is preferably from about 0.5 to about 1.2 kgf/cm³. Stirring is preferably from about 40 hz to about 45 hz.

In a preferred embodiment, the culture medium used for scheduling the culture of the three *Bacillus* and/or fermentation for 100 mL, 1L and 10 L scales is as described in table 1.

Formation of endospores by *Bacillus* cells is induced by an osmotic stress modality imposed during or after the fermentation process of cell multiplication. Osmotic stress is simulated by adding a concentrated salt solution comprising 50 to 400 g/L of Ca(NO₃)₂, 1.0 to 10 g/L of MnCl₂ and 0.1 to 0.8 g/L of FeSO₄, which, in combination, trigger a series of physiological responses in cells, resulting in the induction of endospore formation. Formation of endospores takes place in the stationary phase of bacterial growth, when microorganisms understand stress as an unfavorable condition for survival. It takes place in the end of cell division, mitosis, and comprises the following steps; DNA replication and axial nucleoid formation, core formation through the concentration of cytoplasmic proteins and RNA around the axial nucleus; formation of the endospore septum by the plasma membrane; biosynthesis of peptidoglycans that are deposited between the lipid bilayer comprising a laminar layer designated as the cortex; deposition of protein layers around the cortex by the mother cell, waterproofing the resistance form with the formation of a spore layer and end of the process.

Endospores are known resistance structures for Gram positive bacterial cells. Using the process according to the present invention a sporulation with high efficiency can be achieved, at a rate greater than 90% and concentrations greater than 10⁸ endospores/mL, regardless of random factors.

Preferably, osmotic stress is induced by incubating the cells in a medium containing a saline concentration of 1 g/L to about 15 g/L. Preferably, the salts are Ca(NO₃)₂, MnCl₂ and FeSO₄. More preferably, a solution as described in Table 4 is added to the culture and/or fermentation broth at a ratio of about 1 spore-forming saline to about 36 *Bacillus* culture broth. Preferably, the cells are incubated with the saline solution for about 24 to about 168 hours.

In an alternative embodiment, the present invention provides a product comprising a composition according to the present invention as a plant, in particular, grass, growth promoter.

The present invention further provides the use of the compositions according to the present invention for controlling nematodes and/or for promoting plant growth in agricultural crops.

The present invention further provides methods of controlling nematodes and/or promoting plant growth in agricultural crops comprising the use of a composition according to the present invention in agricultural crops. In a preferred embodiment, the methods of the present invention are applied to grass crops, preferably maize.

Preferably, an agricultural composition according to the present invention is applied to seeds and/or in the planting furrow.

The following examples are intended to illustrate, but not to limit, the invention.

### EXAMPLES

### Example 1 - Crop scheduling

*Bacillus* species are inoculated separately in flasks containing 100 mL of culture medium as described in Table 1 for *B*. *amyloliquefacies* and *B. velezensis* and Table 3 for *B*. *thurigiensis,* being incubated in a 80-200 rpm orbital shaker at 22-38 °C for about 8 to 48 hours. The next scheduling step consists of inoculating stainless-steel flasks containing 1 L of culture medium (Table 1 and Table 3), in which the species are grown separately and incubated for about 8 to 48 hours, with an air flow rate of 0.25 to 1.0 Nm³/h (= 4.16 - 16.67 vvm) and a temperature of about 22 to 38 °C. After this time, the culture is inoculated into stainless steel flasks containing 10 L of culture medium and incubated for about 18 to 96 hours, with an air flow rate of 0.25 to 1.5 Nm³/h (= 0.41 - 2.5 vvm) and a temperature ranging from 22 to 38 °C.

After this time, each culture containing two stainless steel flasks containing 10 L of culture medium is inoculated into a tank containing about 180 L of culture medium specific to each microorganism, Table 2 showing the specific culture medium for *B*. *amyloliquefaciens* and *B. velezensis;* and Table 3 the culture medium specific for *B. thuringiensis* with the addition of a stainless steel flask containing about 5 L of the solution of endospore-forming salts for *Bacillus* spp. (Table 4) and incubated for about 24 to 168 hours, with an air flow rate of 3.0 to 10.0 Nm³/h (= 0.25 - 0.83 vvm) and a temperature ranging from 22 to 38 °C.

**Table 1. Culture medium used for growing B. amyloliquefaciens and B. velezensis up to the 10L scale.**

| | ***Reagents*** | | 1 **L** |
|---|---|---|---|
| **01** | K₂HPO₄ | | 0.1 to 4 g |
| **02** | KH₂PO₄ | | 0.1 to 4 g |
| **03** | MgSO₄.7H₂O | | 0.1 to 0.6 g |
| **04** | NaCl | | 0.05 to 0.3 g |
| **05** | Yeast extract | | 0.1 to 4 g |
| **06** | Peptone | | 0.2 to 4 g |
| **07** | 10% FeCl₃ solution | | 0.05 to 1 mL |
| **08** | 10% MnSO₄ solution | | 0.05 to 1 mL |
| **09** | Sucrose | | 5 to 10 g |

**Table 2. Culture medium used for growing Bacillus spp. for 200 L tanks.**

| | | ***Reagents*** | | 1 **L** |
|---|---|---|---|---|
| **01** | | Maltose | | 2 to 20 g |
| **02** | | Yeast extract | | 1 to 10 g |
| **03** | | NaCl | | 1 to 10 g |

**Table 3. Culture medium used for growing B. thuringiensis spp. for 200 L tanks.**

| | ***Reagents*** | | **1 L** |
|---|---|---|---|
| **01** | Yeast extract | | 1 to 10 g |
| **02** | Peptone | | 1 to 10 g |
| **03** | Glutamate | | 0.1 to 5 g |
| **04** | (NH₄)₂SO₄ | | 0.1 to 5 g |
| **05** | K₂HPO₄ | | 3 to 6 g |
| **06** | KH₂PO₄ | | 3 to 6 g |
| **07** | MgSO₄.7H₂O | | 0.1 to 1 g |
| **08** | CaCl₂.2H₂O | | 0.1 to 1 g |
| **09** | FeSO₄.7H₂O | | 0.01 to 0.1 g |
| **10** | MnSO₄ | | 0.01 to 0.1 g |
| **11** | CuSO₄ | | 0.001 to 0.1 g |
| **12** | ZnSO₄.7H₂O | | 0.001 to 0.1 g |
| **13** | Glucose | | 1 to 60 g |

**Table 4. Spore-forming solution for the three Bacillus species.**

| | | | *Reagents* | | **1 L** |
|---|---|---|---|---|---|
| **01** | | | Ca(NO₃)2 | | 50 to 400 g |
| **02** | | | MnCl₂ | | 1.0 to 10 g |
| **03** | | | FeSO₄ | | 0.1 to 0.8 g |

### Example 2 - Mixture of Bacillus and Botanical Extract in a bioreactor

For the mixture of three different *Bacillus* and botanical extract in a 2,000 L fermenter, preferably the sterilization process using 1,400 L of water with anti-foaming agent is carried out for about 60 to 120 minutes at a temperature of about 121 °C to about 130 °C. Preferably, sterilization is performed at a pressure of about 1.0 - 2.0 Kgf/cm². The air flow rate is preferably of about 1.0 Nm³/h 0 to about 2.5 Nm³/h (= 0.0085 - 0.021 vvm). The pressure is preferably of about 1.0 to about 1.2 kgf/cm³. Stirring is preferably from about 40 hz to about 45 hz. Preferably, in the stabilization process, the three *Bacillus* species containing about 1 to about 30% of allicin-based botanical extract are then inoculated and mixed with the fermenter, preferably for about 30 to about 120 minutes. Preferably, the final product is filled into gallons or bags, packaging in which the product is stored and marketed.

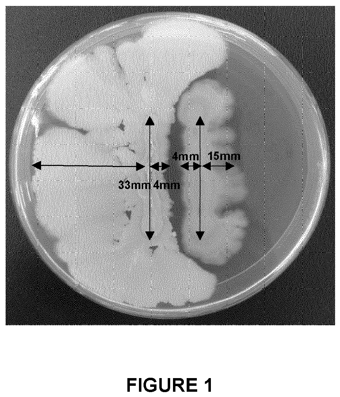

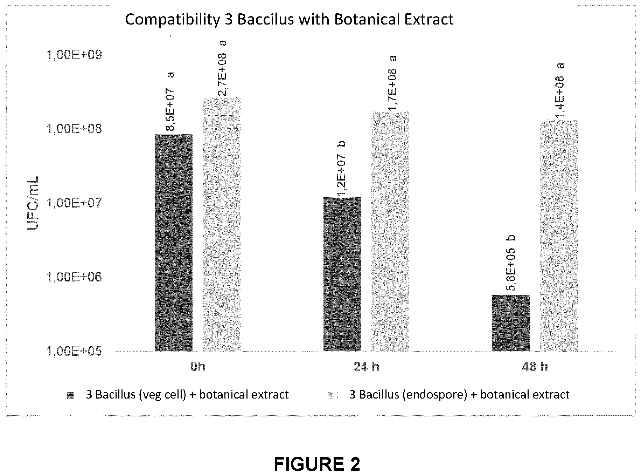

### Example 3 - Induction and stabilization of endospores allows (i) the combination of different Bacillus species (Figure 1) and (ii) their association with botanical extracts (Figure 2).

When garlic botanical extract was added to cells of the three *Bacillus* species, *B. amyloliquefaciens, B. velezensis* and *B. thuringiensis* in their vegetative forms there was a sharp drop in cell viability in just 24 hours, which was even more accentuated after 48 hours. In contrast, when the cells of the species in their respective forms of resistance were subjected to the same conditions, addition of the botanical extract and storage time, great stability of the composition was obtained, without any significant differences in concentration and cell viability. Stability and compatibility achieved in the assay using sporulated cells in a admixture with allicin enables the technology for industrial production and agronomic application.

### Example 4 - Combination of three Bacillus species and botanical extract enhances the nematicide effect on targets of agricultural relevance

In *in vitro* bioassays, mortality of J2 juveniles of *Meloidogyne javanica* nematodes, commonly known as "gall nematodes", or root-knot nematode, has been assessed, being considered one of the most economically relevant groups of phytonemtaoids in the country. J2 stage juveniles are the infective form of nematodes, that is, they are the mobile and active form of the nematode that penetrate into the cell's elongation zone of the root and initiates the process of parasitism.

*Bacillus* spp. are recognized for their nematicide potential thanks to their ability to synthesize a wide range of antibiotics, polypeptides, hydrolytic enzymes and endotoxins that affect the reproductive cycle of nematodes, and act on adult and juvenile forms thereof (Machado et al., 2012). Garlic extract, in turn, has biologically active ingredients, including allicin and polysulfides, which act on insects and nematodes, and has been used as a component of new generation nematicide products (Umetsu and Shirai, 2020).

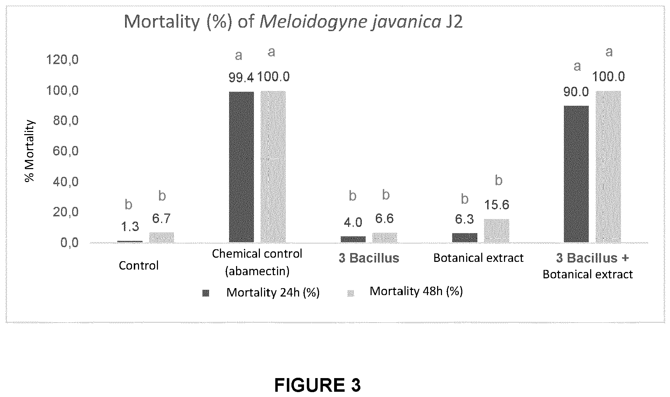

However, as the skilled person can note from Figure 3, the combination of three *Bacillus* species associated with the botanical extract results in a surprising synergistic effect, reaching mortality rates greater than those obtained by using either the *Bacillus* spp. or the botanical extract alone. Such an unexpected effect can be assigned to the combination and potentiation of the mechanisms of action involved in antagonism to nematodes, in such a way that its effect is greater than the sum of the effects of each of the agents applied alone. This surprising effect was only possible thanks to the present invention, which made it possible to mix three different *Bacillus* species (in their endospore forms) with the allicin-rich botanical extract.

Advantageously, the invention acted on the mobile forms of the nematode (J2), which have a greater strength to infect the roots, showing the huge potential of this technology in the management of such a great challenge of current agriculture.

### Example 5 - In addition to enhancing the nematicide effect, the combination of three Bacillus species and botanical extract acts synergistically in promoting plant growth.

Although irrefutable indications exist of the nematicide effect of garlic extract, there is evidence showing that some concentrations of this botanical extract can negatively affect the growth and microbiota of some culture crops (Adeleke et al., 2016).

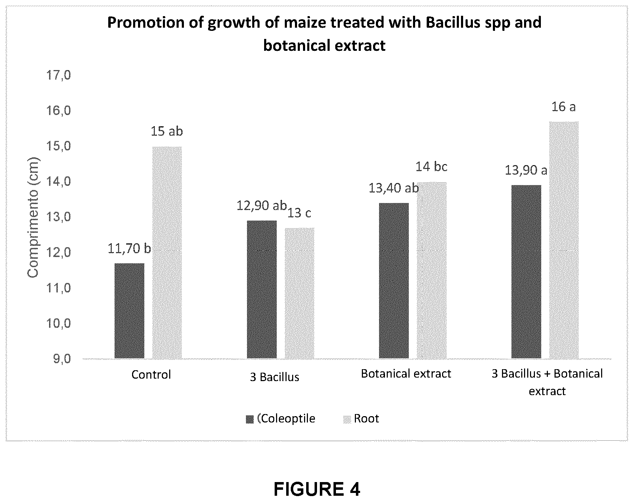

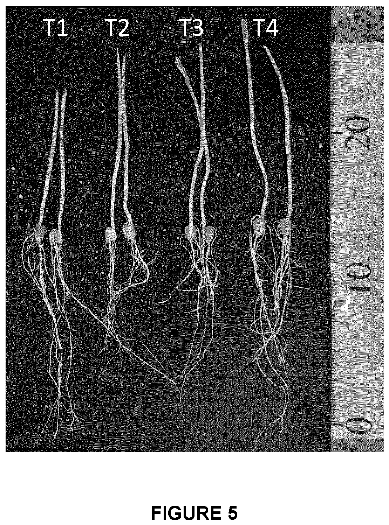

Unexpectedly, the present invention, which consists of the combination of three *Bacillus* species and garlic extract, has promoted plant growth, as can be seen in figures 4 and 5. As observed for the nematicide effect, a synergistic activity of the components of the present invention was verified, so that its effect is greater than the sum of the effects of each of the agents applied alone.

### REFERENCES

ADELEKE, M. T. V. Effect of Allium sativum (garlic) extract on the growth and nodulation of cowpea (Vigna unguiculata) and groundnut (Arachis hypogea). African Journal of Agricultural Research, v. 43, n.11 4304-4312, 2016.
ADETOMIWA, A.A., DU, T.L. AND OLUBUKOLA, O.B.. Bacillus velezensis: phylogeny, useful applications, and avenues for exploitation. Applied Microbiology and Biotechnology, 103, 2019, 3669-3682.
ADJALLE, K. D.; VU, K. D.; TYAGI, R. D.; BRAR, S. K.; VALERO, J. R.; SURAMPALLI, R. Y. Optimization of spray drying process for Bacillus thuringiensis fermented wastewater and wastewater sludge. Bioprocess and Biosystems Engineering, v. 34, n. 2, p. 237-246, 2010.
BOZA, Y.; BARBIN, D.; SCAMPARINI, A.R.P. Effect of spray-drying on quality of encapsulated cells of Beijerinckia sp. Process Biochem, v. 39, p. 1275-1284, 2004.
DE BOER, M.; VAN DER SLUIS, I.; VAN LOON, L.C.; BAKKER, P.A.H.M. Combining fluorescent Pseudomonas spp. strains to enhance suppression of fusarium wilt of radish. European Journal of Plant Pathology, v. 105, p. 201-210, 1999.
BRAVO, A.; GILL, S. S.; SOBERON, M. Mode of action of Bacillus thuringiensis Cry and Cyt toxins and their potential for insect control. Toxicon, v. 49, n. 4, p. 423-435, 2007.
CURTIS, H.; NOLL, U.; STÖRMANN, J.; SLUSARENKO, A. J. Broad-spectrum activity of the volatile phytoanticipin allicin in extracts of garlic (Allium sativum L.) against plant pathogenic bacteria, fungi and Oomycetes. Physiological and Molecular Plant Pathology, v. 65, n. 2, p. 79-89, 2004
DUFFY, B.K.; SIMON, A.; WELLER, D.M. Combination of Trichoderma koningii with fluorescent pseudomonads for control of take-all on wheat. Phytopathology, v. 86, n. 2, p. 188-194, 1996.
Embrapa Soja, 2020. Available at: https://blogs.canalrural.com.br/embrapasoja/2020/05/22/nematoides-um-problema-subestimado-na-soja/; acesso em 13/10/2020.
ERRINGTON, J. Regulation of endospore formation in Bacillus subtilis. Nature Reviews Microbiology, v.1, n.2, p.117-126, 2003.
FUJISAWA, H.; SUMA, K.; ORIGUCHI, K.; KUMAGAI, H.; SEKI, T.; ARIGA, T. Biological and Chemical Stability of Garlic-Derived Allicin. Journal of Agricultural and Food Chemistry, v. 56, n.11, p. 4229-4235, 2008.
GOMES, A. M. A.; MARIANO, R. L. R.; SILVEIRA, E. B.; MESQUITA, J.C.P. Isolamento, seleção de bactérias e efeito de Bacillus spp. na produção de mudas orgânicas de alface. Horticulture Brasileira, v. 21, p. 699-703, 2003.
IDRIS, E. et al. Tryptophan-dependent production of indole-3-acetic acid (IAA) affects level of plant growth promotion by Bacillus amyloliquefaciens FZB42. Molecular plant-microbe interactions: MPMI, v. 20, n. 6, p. 619-626, 2007.
JOHNSON, J.A.C.; ETZEL, M.R. Inactivation of lactic acid bacteria during spray-drying. Aiche Sympos v.89, p. 89-107, 1993.
LEONTIEV, R., HOHAUS, N., JACOB, C.; MARTIN, C.; GRUHLKE, H.; SLUSARENKO, A. J. A Comparison of the Antibacterial and Antifungal Activities of Thiosulfinate Analogues of Allicin. Scientific Report 8, 2018.
LIU, Z. et al. The highly modified microcin peptide plantazolicin is associated with nematicidal activity of Bacillus amyloliquefaciens FZB42. Applied Microbiology and Biotechnology, v. 97, n. 23, p. 10081-10090, 2013.
MACHADO, V.; BERLITZ, D. L.; MATSUMURA, A. T. S.; SANTIN, R. C. M.; GUIMARÃES, A.; SILVA, M. E.; FIUZA, L. M. Bactérias como agentes de controle biológico de fitonematóides. Oecologia Australis, Rydalmere, v. 16, n. 2, p. 165- 182, 2012.
NORIHARU UMETSU, YUICHI SHIRAI. Development of novel pesticides in the 21st century. Journal of Pesticide Science. 2020, Vol.45, No.2, p.54.
RABBEE, M.F. et al., Bacillus velezensis: A Valuable Member of Bioactive Molecules within Plant Microbiomes. Molecules, 24, 2019, 1046. doi:10.3390/molecules24061046
SANSINENEA, E. Discovery and Description of Bacillus thuringiensis. In: Bacillus thuringiensis Biotechnology. [s.l.] Springer, 2012. v. 9789400730p. 1-392.
SCHÜNEMANN, R.; KNAAK, N.; FIUZA, L. M. Mode of Action and Specificity of Bacillus thuringiensis Toxins in the Control of Caterpillars and Stink Bugs in Soybean Culture. ISRN Microbiology, v. 2014, p. 1-12, 2014.
ZAHIRA M. SALEEM; KHALAF S. AI-DELAIMY. Inhibition of Bacillus cereus by Garlic Extracts. Journal of Food Protection, v. 45, n. 2, p. 1007-1009, 1982.
XU, Z. et al. Contribution of bacillomycin D in Bacillus amyloliquefaciens SQR9 to antifungal activity and biofilm formation. Applied and Environmental Microbiology, v. 79, n. 3, p. 808-815, 2013.
XUEYONG, Z.; JIAMPING, D.; JIANBAO, G.; ZINIU, Y. Activity-loss characteristic of spores of Bacillus thuringiensis during spray drying. Food Bioprod Process v. 86, p.37-42, 2008.

## Claims

1. An agricultural composition, **characterized by** comprising endospores of two or more bacterial species from the genus *Bacillus* and a botanical extract comprising allicin.

2. The agricultural composition according to claim 1, **characterized by** comprising endospores of three or more bacterial species of the genus *Bacillus.*

3. The agricultural composition according to claim 1, **characterized in that** bacteria of the genus *Bacillus* are selected from the group consisting of *B. amyloliquefaciens, B. thuringiensis* and *B. velezensis.*

4. The agricultural composition according to claim 1, **characterized in that** the botanical extract comprising allicin is garlic extract.

5. The agricultural composition according to claim 1, **characterized in that** it further comprises additional components that promote the germination of endospores in metabolically active forms only when applied in the field.

6. The process of manufacturing an agricultural composition, **characterized by** comprising:
(a) fermentation of microbial cultures of two or more species of the genus *Bacillus;*
(b) imposing osmotic stress during or after the fermentation process; and
(c) formulation of an agricultural composition comprising the mixture of bacterial cells with the botanical extract comprising allicin in the same container.

7. The process according to claim 1, **characterized in that** three or more *Bacillus* species are used.

8. The process according to claim 1, **characterized in that** the three *Bacillus* species are selected from the group consisting of *Bacillus amyloliquefaciens, Bacillus velezensis* and *Bacillus thuringiensis.*

9. The process according to claim 1, **characterized in that** induction of the formation of endospores of step (a) takes place in the tank environment.

10. The process according to claim 1, **characterized in that** fermentation of the culture is by batch fermentation.

11. The process according to claim 1, **characterized in that** the process of stabilizing the product of step (b) is carried out for about 60 to about 120 minutes.

12. The process according to claim 1, **characterized in that** the process of inducing the formation of endospores of step (a) is carried out at a temperature of about 22 °C to about 37 °C.

13. The process according to claim 1, **characterized in that** the process of inducing the formation of endospores of step (a) is carried out at an air flow rate of about 3.0 Nm³/h (0.25 vvm) to about 10.0 Nm³/h (= 0.83 vvm).

14. The process according to claim 1, **characterized by** further comprising the sequential expansion of the culture of different *Bacillus* spp. for inoculation of the fermentation culture.

15. The process according to claim 1, **characterized in that** the *Bacillus* species are inoculated separately.

16. The process according to claim 1, **characterized in that** the sequential expansion is carried out at volumes of about 100 mL to about 10 L, about 180 L to about 2,000 L.

17. A process **characterized in that** the *Bacillus* species are expanded by incubation in an orbital shaker at about 80 rpm to about 200 rpm.

18. The process according to claim 11, **characterized in that** the *Bacillus* species are expanded by incubation for about 8 hours to about 48 hours.

19. The process according to claim 12, **characterized in that** the *Bacillus* species are expanded in stainless steel flasks containing about 1 L of culture medium and about 10 L of culture medium.

20. The process according to claim 13, **characterized in that** the species are incubated for about 8 to about 48 hours.

21. The process according to claim 14, **characterized in that** the strains are incubated at an air flow rate of about 0.25 Nm³/h to about 1.0 Nm³/h (= 4.16 - 16.67 vvm) for 1L of culture medium and an air flow rate of about 0.25 Nm³/h to about 1.0 Nm³/h (= 0.41 - 1.67 vvm) for 10 L of culture medium.

22. The process according to any one of claims 13 to 16, **characterized in that** after segregated cultivation of the species in two stainless steel flasks of about 10 L, said two flasks are inoculated into tanks containing about 180 L of culture medium.

23. The process according to claim 16, **characterized in that** the strains are incubated for about 24 to about 168 hours.

24. The process according to claim 16, **characterized in that** the strains are incubated at an air flow rate of about 1.0 to about 15.0 Nm³/h (= 0.16 - 1.25 vvm).

25. The process according to any one of claims 1 to 18, **characterized in that** the incubation temperature is of about 27 °C to about 37 °C.

26. The process according to claim 1, **characterized in that** the fermentation step is carried out at a pressure of about 1.0 to about 2.0 kgf/cm³.

27. The process according to claim 1, **characterized in that** the fermentation step is carried out with stirring of about 40 hz to about 45 hz.

28. The process **characterized in that** the fermentation step is carried out at a temperature of about 27 °C to about 37 °C.

29. The process according to claim 1, **characterized in that** the fermentation step is carried out at an air flow rate of about 1.0 Nm³/h to about 2.5 Nm³/h (= 0.0085 - 0.021 vvm).

30. The process according to claim 1, **characterized in that** the step of mixing *Bacillus* spp. and botanical extract is carried out for about 30 to about 120 minutes.

31. A composition, **characterized in that** it is obtained by the process, as defined in any one of claims 1 to 26.

32. The use of a composition as defined in claim 1, **characterized in that** it is for application in agricultural crops.

33. Use of a composition as defined in claim 1, **characterized in that** it is for application on seeds or sowing furrows.

34. A method of controlling nematodes in agricultural crops, **characterized in that** it comprises the application of a composition as defined in claim 1 to the agricultural crop.

35. A method of promoting plant growth in agricultural crops, **characterized in that** it comprises the application of a composition as defined in claim 1 to the agricultural crop.

36. Invention, **characterized by** any of its embodiments or any applicable claim categories, for example, product, process or use, encompassed by the subject matter as initially disclosed, described or illustrated in the patent application.
